# EUROPÄISCHE PATENTSCHRIFT

(11) **EP 0 910 797 B1**
(45) Veröffentlichungstag und Bekanntmachung des Hinweises auf die Patenterteilung: **02.01.2002**
(21) Anmeldenummer: 97929306.5
(22) Anmeldetag: 28.06.1997
(51) Int. Cl.: G01N 33/28

(54) **VORRICHTUNG ZUR ÜBERWACHUNG VON GEBRAUCHSEIGENSCHAFTEN VON FLUIDEN, INSBESONDERE VON DRUCKFLÜSSIGKEITEN IN FLUIDTECHNISCHEN ANLAGEN**
DEVICE FOR MONITORING THE USE PROPERTIES OF FLUIDS, IN PARTICULAR PRESSURE FLUIDS IN FLUID-TECHNICAL INSTALLATIONS
DISPOSITIF POUR SURVEILLER LES PROPRIETES D'UTILISATION DE FLUIDES, EN PARTICULIER DE LIQUIDES SOUS PRESSION DANS DES INSTALLATIONS HYDRAULIQUES

(30) Priorität: 09.07.1996 DE 19627587
(43) Veröffentlichungstag der Anmeldung: 28.04.1999
(73) Patentinhaber: Hydac Filtertechnik GmbH, 66280 Sulzbach/Saar (DE)
(72) Erfinder: SCHÖN, Otmar, D-66450 Bexbach (DE); TUMBRINK, Manfred, D-64732 Bad König (DE); KIRSCH, Bernhard, D-66399 Mandelbachtal (DE)
(74) Vertreter: Patentanwälte Bartels und Partner
(86) Internationale Anmeldenummer: EP9703393
(87) Internationale Veröffentlichungsnummer: WO9801750

(56) Entgegenhaltungen:
- EP-A- 0 435 713
- WO-A-90/06500
- PATENT ABSTRACTS OF JAPAN vol. 13, no. 108 (P-843), 15.März 1989 & JP 63 285467 A (TOSHIBA CORP), 22.November 1988,

## Beschreibung

Die Erfindung bezieht sich auf eine Vorrichtung mit den Merkmalen des Oberbegriffs des Anspruches 1.

Beim Betrieb fluidtechnischer Anlagen ist die Überwachung bestimmter Eigenschaften der Druckflüssigkeit für die Beurteilung von deren Gebrauchsfähigkeit und für die Steuerung und Überwachung von Aggregaten und Einrichtungen von großer Bedeutung, die zur Aufrechterhaltung der Gebrauchsfähigkeit im Betrieb dienen.

Gebrauchseigenschaften des Fluides, bei denen das Unter- oder Überschreiten von Grenzwerten die Einsatzfähigkeit einer fluidtechnischen Anlage im Betrieb beeinträchtigen kann, sind unter anderen:
a) Verschmutzung des Fluides mit Feststoffen;
b) Schmiereigenschaften des Fluides;
c) Verunreinigungen des Fluides mit anderen, die Korrosion fördernden Substanzen, insbesondere mit Wasser;
d) Gehalt des Fluides an durch Oxidation entstandenen Abbauprodukten, die die Gebrauchsfähigkeit beeinträchtigen (Alterung);
e) Versorgung der Pumpen und/oder Aggregate mit Fluid, d.h. der Füllstand im Fluidbehälter (der Füllstand wird im vorliegenden Rahmen als eine Gebrauchseigenschaft des Fluides betrachtet).

Falls im Betrieb ein Zustand eintritt, der die Funktion der betreffenden Anlage gefährdet, sind entweder selbsttätig Maßnahmen zu treffen, um das Problem zu beseitigen (z.B. Einschalten eines Kühleraggregates), oder der Betrieb der Anlage muß zur Vermeidung von Folgeschäden eingeschränkt oder eingestellt werden. In jedem Falle ist es daher wesentlich, daß der Betreiber über den Eintritt einer Störung informiert wird, daß eine Protokollierung stattfindet und gegebenenfalls eine Alarmgabe erfolgt.

Wenn zur Überwachung der Gebrauchseigenschaften von Fluiden eine Probenentnahme auf mehr oder weniger automatisierte Weise durchgeführt und eine Analyse des Probevolumens im Labor vorgenommen wird, gelangt man nur zu einer unvollständigen Beurteilung der Gebrauchseigenschaften, weil durchgeführte Analysen und Messungen nur dann miteinander vergleichbar sind, wenn die Meßgrößen an den verschiedenen Meßorten, d.h. den Orten der Probenentnahmen, in einem definierten räumlichen und zeitlichen Zusammenhang zueinander stehen. Bei zwei zeitlich verschiedenen Probenentnahmen kann nicht garantiert werden, daß der Meßort der gleiche ist. Eine zeitgleiche Messung zweier Größen an verschiedenen Orten im System ist nur aussagekräftig, falls keine Gradienten vorliegen, beispielsweise Druck- und Temperaturgradienten. Zur Vermeidung dieser Probleme ist es aus einer in dem Dokument JP-A-63285467 aufgezeigten Überwachungsvorrichtung der eingangs genannten Art bekannt, die Sensoren mit der Fluidpumpe in Reihe zu schalten, so daß sie von demselben Fluidstrom durchströmbar sind, um dadurch aufgrund einer Probeentnahme an einer einzigen Meßstelle eine kontinuierliche Ermittlung unterschiedlicher Meßgrößen anhand eines und desselben Fluidstromes, der mehrere Sensoren passiert, zu gewährleisten. Dadurch ist eine weitestgehend fehlerfreie Überwachung der für die Einsatzfähigkeit relevanten Eigenschaften des Fluides möglich.

Der Erfindung liegt die Aufgabe zugrunde, eine Vorrichtung dieser Art dahingehend auszugestalten, daß sie sich durch eine einfache und besonders kompakte Bauweise auszeichnet. Diese Aufgabe wird erfindungsgemäß durch eine Vorrichtung mit den Merkmalen des Anspruches 1 gelöst.

Dadurch, daß die gesamte Prüfeinrichtung an einem Rahmen zu einer Einheit zusammengefaßt ist, die, einschließlich der Sensoranschlußplatine mit der die Sensorsignale verarbeitenden Elektronik, für in das Fluid eingetauchte Betriebsweise ausgebildet und unmittelbar in den Fluidbehälter einbaubar ist, lassen sich die für die Überwachung erforderlichen Meßgrößen mit hoher Genauigkeit und bei kleinstmöglichem Raumbedarf der Vorrichtung ermitteln.

Vorzugsweise enthält die vom Proben-Fluidstrom durchströmte Reihenschaltung im Sensorblock einen Partikelsensor mit integrierter Trübungsmessung zur Ermittlung der Partikelkonzentration, ein Viskosimeter und einen Feuchtesensor mit integriertem NTC-Widerstand, um sowohl den Gehalt an freiem Wasser als auch die Temperatur des Fluides zu ermitteln.

Bei Verwendung eines Viskosimeters in Form eines Reibradviskosimeters mit integrierter Bremsspule eröffnet sich die vorteilhafte Möglichkeit, nicht nur die Viskosität des Fluides, sondern auch die Strömungsrate des Proben-Fluidstromes zu ermitteln, was unter anderem auch eine Messung durchflußempfindlicher Meßgrößen und eine Funktionskontrolle der Überwachungsvorrichtung ermöglicht.

In besonders vorteilhafter Weise kann dadurch, daß die Sensoranschlußplatine mit der die Sensorsignale verarbeitenden Elektronik in das Fluid eingetaucht ist, eine Ermittlung der Dielektrizitätskonstante des Fluides mittels eines an der Platine vorgesehenen, unmittelbar in Fluidkontakt befindlichen Interdigitalkondensators erfolgen. Die Korrosionswirkung des Fluides auf Kupfer kann mit Hilfe eines Interdigitalkondensators aus Kupfer-Leiterbahnen abgeleitet werden.

Die von der Elektronik erfaßten Sensorsignale werden vorzugsweise digitalisiert und in busfähige Primärdaten umgewandelt, die von beliebigen Datenendgeräten erfaßt und verarbeitet werden können. Eine derartige Anordnung eignet sich in besonderer Weise zur unmittelbaren Steuerung von Hilfsaggregaten zur Beeinflussung des Zustandes des Fluides, beispielsweise
Nebenstromkühler, Heizelemente,
Nebenstromfilter zum Entfernen von Feststoffen,
Nebenstromfilter zum Entfernen von Wasser,
Spül- und Reinigungspumpen.

Nachstehend ist die Erfindung anhand eines in der Zeichnung dargestellten Ausführungsbeispieles im einzelnen erläutert.

Es zeigen:
- Fig.1: eine vereinfachte Diagrammdarstellung des hydraulischen Schaltplanes eines Ausführungsbeispieles der erfindungsgemäßen Überwachungsvorrichtung;
- Fig.2: einen stark schematisch vereinfacht, teils aufgebrochen und abgebrochen gezeichneten Vertikalschnitt des Ausführungsbeispieles der Vorrichtung und
- Fig.3: einen Querschnitt des Ausführungsbeispieles, im wesentlichen entsprechend der Schnittlinie III-III von Fig.2.

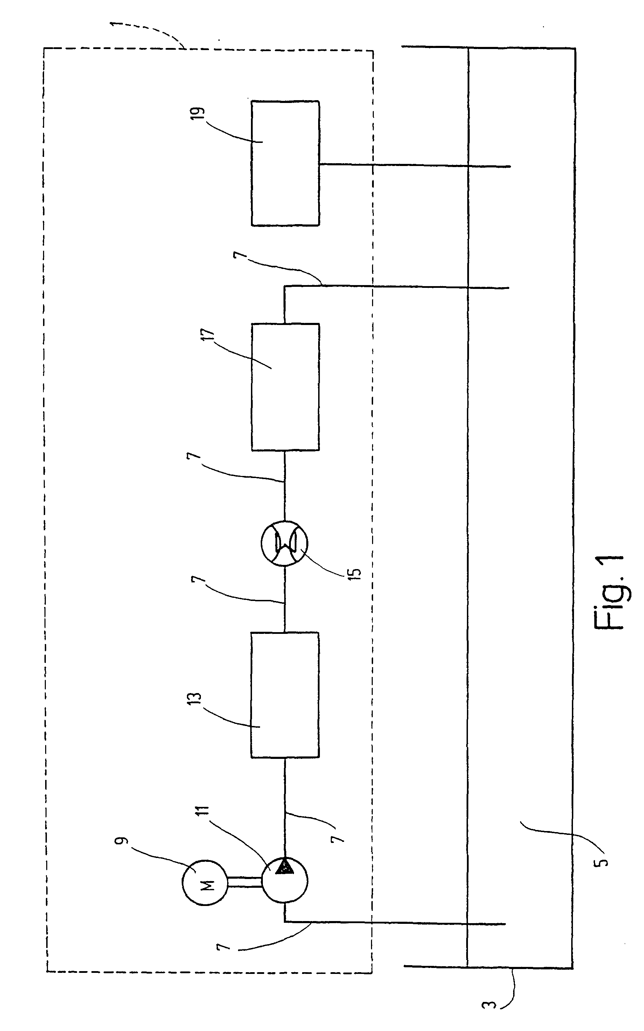

Eine in Fig.1 mit gestrichelter Linie angedeutete Baueinheit 1, in der die wichtigsten Komponenten der hier zu beschreibenden Überwachungsvorrichtung zusammengefaßt sind, ist für den Einbau in einen Tank 3 vorgesehen, der eine Druckflüssigkeit 5 für eine nicht gezeigte fluidtechnische Anlage enthält. Die Baueinheit 1 enthält Strömungskanäle 7, die eine hydraulische Reihenschaltung bilden, um mittels einer durch einen Elektromotor 9 angetriebenen Pumpe 11 einen Proben-Fluidstrom nacheinander durch einen Partikelsensor 13, einen Reibradsensor 15 mit integrierter Bremsspule 43 sowie einen Feuchtesensor 17 hindurchströmen zu lassen. Außerdem ist in Fig.1 ein Drucksensor 19 gezeigt, der als in das Fluid 5 eingetauchter Drucksensor zur Ermittlung der Füllstandshöhe im Tank 3 dient.

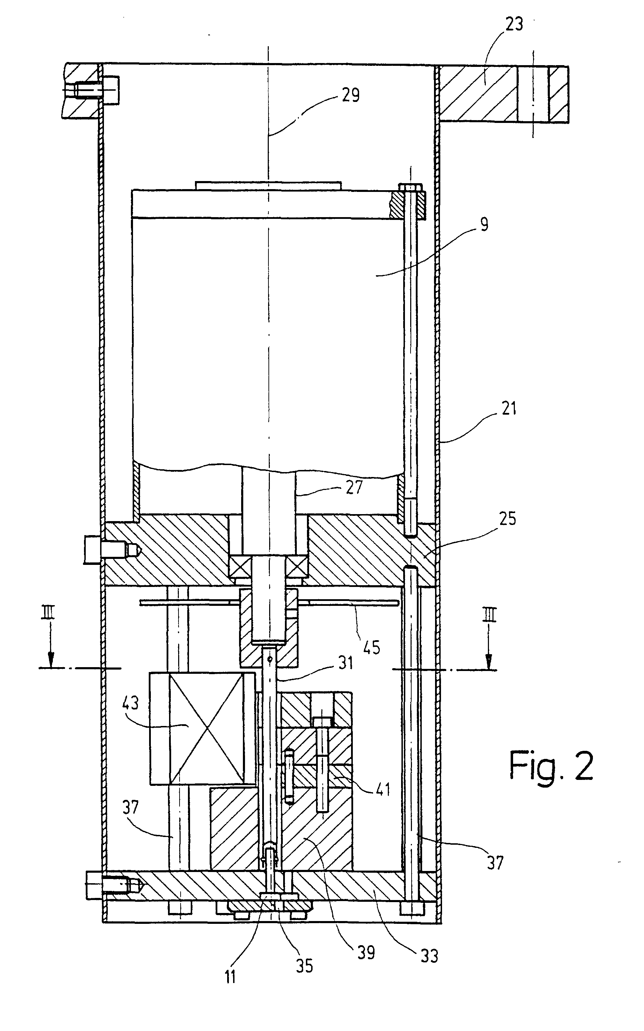

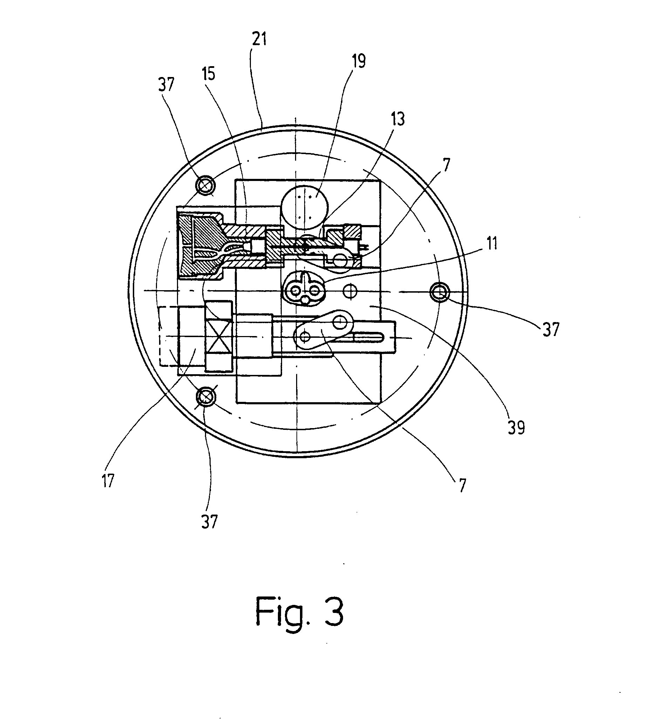

Wie Fig.2 und 3 zeigen, weist die Baueinheit 1 einen rohrförmigen Rahmen 21 auf, der mittels eines an seinem oberen Endbereich angebrachten Befestigungsflansches 23 mit dem Rand einer Öffnung in der oberen Wand eines betreffenden Tanks 3 verschraubbar ist, so daß der Rahmen 21 in die im Tank 3 befindliche Druckflüssigkeit 5 eintaucht. Beim Ausführungsbeispiel ist der Rahmen 21 durch ein starkes Drahtgeflecht gebildet, stellt also ein für den Zutritt des Fluides 5 offenes Bauteil dar. Der dem Antrieb der Pumpe 11 dienende Motor 9, der als Tauch- oder Unterölmotor ausgebildet ist, ist mit seinem Lagerschild 25 etwa auf mittlerer Höhe des rohrförmigen Rahmens 21 mit diesem so verschraubt, daß sich seine Motorwelle 27 koaxial zur Längsachse 29 des Rahmens 21 erstreckt. Eine Verlängerung 31 der Motorwelle 27 ist mit der Antriebsseite der Pumpe 11 gekuppelt, bei der es sich um eine Zahnradpumpe handelt, die in einer Fußplatte 33 am unteren Endbereich des rohrförmigen Rahmens 21 sitzt und mit ihrer saugseitigen Eintrittsöffnung 35 in die im Tank 3 befindliche Druckflüssigkeit 5 eintaucht.

Dank der Verschraubung der Fußplatte 33 mit dem Lagerschild 25 über Gewindebolzen 37 ergibt sich, ungeachtet dessen, daß der rohrförmige Rahmen 21 als Drahtgeflecht ausgebildet ist, eine mechanisch steife Struktur.

Ein auf der Oberseite der Fußplatte 33 angebrachter Sensorblock 39 trägt die in Fig.1 gezeigten Sensoren, nämlich den Partikelsensor 13, den Reibradsensor 15, den Feuchtesensor 17 und den Drucksensor 19. Außerdem bildet der Sensorblock 39 zusammen mit einer Zwischenplatte 41 in seinem Inneren die Strömungskanäle 7, um, ausgehend von der Druckseite der Zahnradpumpe 11, die hydraulische Reihenschaltung für den Fluidstrom durch die Sensoren 13, 15 und 17 herzustellen.

Beim vorliegenden Ausführungsbeispiel ist als Partikelsensor 13 ein Partikelzähler der in der EP 0 427 908 B1 bekannten Art vorgesehen. Der als Viskosimeter dienende Reibradsensor 15 ist von der aus der EP 0 446 246 B1 bekannten Art mit einer Bremsspule 43, die wahlweise aktivierbar und deaktivierbar ist. Bei deaktivierter Bremsspule 43 kann dieser Sensor auch zur Ermittlung der Strömungsrate dienen.

Als Feuchtesensor 17 ist beim Ausführungsbeispiel ein Sensor vorgesehen, der von der Firma Michell Instruments unter der Bezeichnung TDT 300 vertrieben wird und der einen integrierten Thermistor (NTC-Widerstand) zur Temperaturermittlung aufweist.

Bei dem Drucksensor 19 handelt es sich um einen piezoresistiven Sensor, wie er von der Firma Alcatel SEL unter der Bezeichnung DS1 vertrieben wird.

Eine unterhalb des Lagerschildes 25 angebrachte Sensoranschlußplatine 45 trägt die zur Auswertung und Verarbeitung der Sensorsignale vorgesehene Elektronik. Diese ist, ebenso wie der als Tauchmotor ausgebildete Elektromotor 9, für in die Druckflüssigkeit 5 eingetauchten Betrieb ausgelegt. Zur Elektronik gehörende Interdigitalkondensatoren zur Ermittlung der Dielektrizitätskonstante der Druckflüssigkeit 5 und deren Korrosionswirkung auf Kupfer stehen somit mit der Druckflüssigkeit 5 in unmittelbarer Berührung.

Die Elektronik der Sensoranschlußplatine 45 liefert busfähige Daten zur Betriebssteuerung der betreffenden fluidtechnischen Anlage, beispielsweise über einen PC oder einen Mikroprozessor als Prozeßrechner.

## Patentansprüche

1. Vorrichtung zur Überwachung von Gebrauchseigenschaften von Fluiden (5), insbesondere von Druckflüssigkeiten in fluidtechnischen Anlagen, mit einer Prüfeinrichtung, mittels deren ein Probevolumen des Fluides (5) aus einem dieses enthaltenden Behälter (3) entnehmbar und einem Sensorgerät zuführbar ist, das für die Ermittlung unterschiedlicher, jeweils eine Gebrauchseigenschaft des Fluides darstellender Meßgrößen eine Mehrzahl von Sensoren (13,15,17) aufweist, die mit der Fluidpumpe (11) in Reihe geschaltet und von demselben Fluidstrom durchströmbar sind, **dadurch gekennzeichnet, daß** die Sensoren (13,15,17) zusammen mit der Fluidpumpe (11) und mit die Reihenschaltung bildenden Fluid-Strömungskanälen (7) zu einem Sensorblock (39) integriert sind, daß der Sensorblock (39) mit der Fluidpumpe (11) sowie ein diese antreibender Motor (9) an einem Rahmen (21) befestigt sind, der durch eine Öffnung des Fluidbehälters (3) in diesen einführbar und als für den Zutritt des Fluides (5) offenes Bauteil, vorzugsweise rohrartiges Bauteil, ausgebildet ist, daß der Motor (9) und die Sensoren (13,15,17) für in das Fluid (5) eingetauchte Betriebsweise ausgebildet sind und daß auch eine Sensoranschlußplatine (45), die innerhalb des Rahmens (21) angeordnet ist und eine die Sensorsignale verarbeitende Elektronik trägt, für in das Fluid (5) eingetauchte Betriebsweise ausgebildet ist.

2. Vorrichtung nach Anspruch 1, **dadurch gekennzeichnet, daß** als mit der Fluidpumpe (11) in Reihe geschaltete Sensoren zumindest ein Partikelsensor (13) und ein Viskosimeter (15) vorgesehen sind.

3. Vorrichtung nach Anspruch 2, **dadurch gekennzeichnet, daß** die Reihenschaltung der Sensoren zusätzlich einen Feuchtesensor (17) aufweist.

4. Vorrichtung nach Anspruch 3, **dadurch gekennzeichnet, daß** ein als Temperatursensor dienender Feuchtesensor (17) mit integriertem Thermistor vorgesehen ist.

5. Vorrichtung nach einem der Ansprüche 2 bis 4, **dadurch gekennzeichnet, daß** als Viskosimeter ein Reibradsensor (15) mit Bremseinrichtung (43) vorgesehen ist.

6. Vorrichtung nach einem der Ansprüche 1 bis 5, **dadurch gekennzeichnet, daß** die Prüfeinrichtung einen Drucksensor (19) zur Ermittlung des Füllstandes im Fluidbehälter (3) aufweist.

7. Vorrichtung nach einem der Ansprüche 1 bis 6, **dadurch gekennzeichnet, daß** die Sensoranschlußplatine (45) einen Interdigitalkondensator zur Ermittlung der Dielektrizitätskonstante des Fluides (5) aufweist.

8. Vorrichtung nach Anspruch 7, **dadurch gekennzeichnet, daß** die Sensoranschlußplatine (45) zur Ermittlung der Korrosionswirkung des Fluides (5) auf Kupfer einen Interdigitalkondensator aus Kupfer-Leiterbahnen aufweist.

## Claims

1. Device for monitoring the useful properties of fluids (5), in particular pressure fluids in fluid-technical installations, with a test device whereby a sample of the fluid (5) can be removed from the tank (3) holding it and carried to a sensor unit having a plurality of sensors (13, 15, 17) for determining measured variables, each representing a useful property of the fluid, which are connected in series with the fluid pump (11) and overflowable by the same flow of fluid, **characterised in that** the sensors (13, 15, 17) are combined with the fluid pump (11) and the series-connected fluid flow lines (7) to form a sensor block (39), that the sensor block (39) is mounted together with the fluid pump (11) and a motor (9) driving it to a frame (21) which can be introduced into the fluid tank (3) through its opening and is designed as an open component, preferably a tube-like component, for entry of the fluid 5, that the motor (9) and the sensors (13, 15, 17) are designed for immersed operation in the fluid (5) and that a sensor connection board (45) which is arranged within the frame (21) and carries electronics processing the sensor signals is also designed for immersed operation in the fluid (5).

2. Device according to Claim 1, **characterised in that** a particle sensor (13) and a viscosimeter (15) are envisaged as the sensors series-connected with the fluid pump (11).

3. Device according to Claim 2, **characterised in that** the sensors connected in series include an additional moisture sensor (17).

4. Device according to Claim 3, **characterised in that** a moisture sensor (17) with an integral thermistor is envisaged as the temperature sensor.

5. Device according to one of the Claims 2 to 4, **characterised in that** a friction wheel sensor with a braking device (43) is envisaged as the viscosimeter.

6. Device according to one of the Claims 1 to 5, **characterised in that** the testing device has a pressure sensor (19) for determining the fluid level in the tank (3).

7. Device according to one of the Claims 1 to 6, **characterised in that** the sensor connection board (45) has an inter-digital capacitor for determining the dielectric constant of the fluid (5).

8. Device according to Claim 7, **characterised in that** the sensor connecting board (45) has an inter-digital capacitor on copper tracks to determine the effect of the fluid (5) on copper.

## Revendications

1. Dispositif de surveillance des caractéristiques d'emploi des fluides (5), et notamment des fluides sous-pression dans les installations hydrauliques, comprenant un dispositif de contrôle, au moyen duquel un volume test de fluide (5) peut être prélevé dans un réservbir (3) contenant ledit fluide et alimenté depuis un moyen formant capteur, qui montre, pour la détermination des différentes grandeurs mesurées présentant à chaque fois une caractéristique d'emploi du fluide, une majorité de capteurs (13, 15, 17) qui sont raccordés en série avec la pompe de fluide (11) et peuvent être parcourus par le même flux de fluide, **caractérisé en ce que** les capteurs (13, 15, 17) sont intégrés avec la pompe hydraulique (11) et avec le couplage en série des canaux de débit du fluide (7) à un bloc de capteurs (39), de telle façon que le bloc de capteurs (39) avec la pompe hydraulique (11) ainsi qu'un moteur (9) actionnant celle-ci soient attachés à un cadre (21), qui peut être incorporé à celui-ci au moyen d'un évent du réservoir contenant le fluide (3) et qui est composé, comme pour l'accès du fluide (5), d'un élément ouvert, de préférence, d'un élément de type conduit, de telle façon que le moteur (9) et les capteurs (13, 15, 17) sbient appropriés pour un mode de fonctionnement par immersion dans un fluide (5) et que la platine de raccordement des capteurs (45), qui est disposée au sein du cadre (21) et qui supporte un dispositif électronique permettant le traitement des signaux de capteurs, soit appropriée pour un mode de fonctionnement par immersion dans un fluide (5).

2. Dispositif selon la revendication 1, **caractérisé en ce qu'**un capteur de particules (13) et un viscosimètre (15) au moins soient prévus comme capteurs montés en série avec la pompe hydraulique (11).

3. Dispositif selon la revendication 2, **caractérisé en ce que** le montage en série des capteurs comprend en plus un capteur d'humidité (17).

4. Dispositif selon la revendication 3, **caractérisé en ce qu'**un capteur d'humidité (17) agissant comme un capteur de température doté d'une thermistance intégrée.

5. Dispositif selon l'une quelconque des revendications 2 à 4, **caractérisé en ce qu'**un capteur à galet de friction (15) doté d'une bobine de frein intégrée (43) est prévu comme viscosimètre.

6. Dispositif selon l'une quelconque des revendications 1 à 5, **caractérisé en ce que** le dispositif de contrôle présente un capteur de pression (19) servant à déterminer le niveau de remplissage du réservoir hydraulique (3).

7. Dispositif selon l'une quelconque des revendications 1 à 6, **caractérisé en ce que** la platine de raccordement des capteurs (45) présente un condensateur internumérique servant à déterminer la constante diélectrique du fluide (5).

8. Dispositif selon la revendication 7, **caractérisé en ce que** la platine de raccordement des capteurs (45) présente un condensateur intamuniérique en époxy à câblage de cuivre servant à déterminer la corrosion du fluide sur lame de cuivre.
